# EUROPÄISCHE PATENTSCHRIFT

(11) **EP 0 749 733 B1**
(45) Veröffentlichungstag und Bekanntmachung des Hinweises auf die Patenterteilung: **29.05.2002**
(21) Anmeldenummer: 95810417.6
(22) Anmeldetag: 21.06.1995
(51) Int. Cl.: A61F 2/38, F16B 37/04

(54) **Tibiaplattform zu einer Kniegelenkprothese und Kniegelenkprothese mit einer derartigen Tibiaplattform**
Tibialplatform for a knee-joint prosthesis and knee-joint prosthesis with such a tibialplatform
Plateau de tibia pour une prothèse articulaire du genou, et prothèse articulaire du genou comprenant un tel plateau de tibia

(43) Veröffentlichungstag der Anmeldung: 27.12.1996
(73) Patentinhaber: Sulzer Orthopädie AG, 6340 Baar (CH)
(72) Erfinder: Oehy, Jürg, CH-8405 Winterthur (CH); Bider, Kurt, CH-8406 Winterthur (CH); Schoch, Martin, CH-8143 Stallikon (CH); Schwägerl, Wolfgang, Prof. Dr. med., A-1160 Wien (AT); Böhler, Nikolaus, Prof. Dr. med., A-4020 Linz (AT)
(74) Vertreter: Manitz, Finsterwald & Partner Gbr

(56) Entgegenhaltungen:
- EP-A- 0 170 779
- EP-A- 0 495 340
- EP-A- 0 502 815
- GB-A- 1 057 763
- US-A- 5 019 103

## Beschreibung

Die Erfindung betrifft eine Tibiaplattform entsprechend dem Oberbegriff des Patentanspruchs 1.

Ferner betrifft die Erfindung eine mit einer derartigen Tibiaplattform ausgeführte Kniegelenkprothese sowie einen Verschlussstopfen für eine derartige Tibiaplattform.

Eine aus der FR-A-2 653 992 bekannte Tibiaplattform der genannten Art enthält einen metallischen Verankerungsteil und eine in diesen einsetzbare Gelenkauflage aus Polyäthylen. Der Verankerungsteil ist mit in den Tibiaknochen einführbaren Vorsprüngen in Form eines zentralen Zapfens und zweier ringförmiger Ansätze ausgeführt, welch letztere je eine Durchtrittsöffnung zur Aufnahme eines dübelartigen Halteelements bzw. eines mit einer schrägen Führungsbohrung versehenen Klemmteils für eine Knochenschraube enthalten. Für die Führung der Knochenschrauben, die hauptsächlich der Verdrehsicherung des Verankerungsteils auf einer Resektionsebene der Tibia dienen, sind somit relativ voluminöse Bohrungen zur Aufnahme des Dübels und des Klemmteils erforderlich, welche den Tibiaknochen schwächen können. Falls aus irgendwelchen Gründen, z.B. aufgrund des Zustands des Tibia-Knochengewebes, auf die Verwendung der oder einer der Knochenschrauben verzichtet wird, kann zudem - durch Kaltfliessen des Kunststoffs der Gelenkauflage im Bereich der jeweils nicht benutzten Durchtrittsöffnung bzw. Durchtrittsöffnungen - Kunststoffabrieb und damit ein direkter Kontakt zwischen dem Kunststoff der Gelenkauflage und dem Tibia-Knochengewebe entstehen.

Aus der EP-A-0,495,340 ist eine Tibiaplattform zu einer Kniegelenkprothese bekannt, welche ein zum Befestigen an einem Tibiaknochen und zur Aufnahme einer Gelenkauflage bestimmtes Verankerungsteil aufweist. Dieses weist Durchtrittsöffnungen auf, welche je zur Aufnahme eines in den Tibiaknochen einfuhrbaren Bebestigungselements geeignet bzw. bestimmt sind. Die Durchtrittsöffnungen sind jeweils mit einer an der Femurseite der Gelenkauflage ausgebildeten Amsenkung ausgeführt.

Der Erfindung liegt die Aufgabe zugrunde, eine insbesondere in dieser Hinsicht verbesserte Tibiaplattform in einer vereinfachten Ausführung zu schaffen, welche mit geringem Aufwand bei der Vorbereitung des Tibiaknochens eine einfache Anpassung der Befestigungsanordnung an die jeweiligen anatomischen Verhältnisse gestattet, und welche zugleich eine sichere Abschirmung des Knochengewebes gegen unerwünschten Kontakt mit Abriebpartikeln gewährleistet.

Diese Aufgabe wird gemäss der Erfindung durch die im kennzeichnenden Teil des Patentanspruchs 1 angegebenen Merkmale gelöst.

Die erfindungsgemässe Bestückung des Verankerungsteils mit einem oder mehreren, je einem der von Befestigungselementen freien Durchtrittsöffnungen zugeordneten Verschlussstopfen gestattet vor dem Einsetzen der Gelenkauflage eine gezielte, kontrollierbare örtliche Abschirmung der jeweils im Bereich der nicht benutzten Durchtrittsöffnung bzw. Durchtrittsöffnungen befindlichen Partie des Knochengewebes der Tibia. Der erfindungsgemäss ausgebildete Verschlussstopfen ist relativ einfach herstellbar, und es kann ein relativ grosser Vorrat von Verschlussstopfen bereitgestellt werden, die vom Chirurgen je auf einfache Weise in die jeweils freie Durchtrittsöffnung eingesetzt bzw. aus dieser entfernt werden können. Die erfindungsgemässe Ausführung gestattet insbesondere eine von der Form und von der Anbringung der Gelenkauflage unabhängige Abdichtung der Durchtrittsöffnungen in einer vorteilhaft frühen Operationsphase, z.B. vor oder unmittelbar nach dem Aufsetzen des Verankerungsteils auf die Resektionsfläche des Tibiaknochens. Ein weiterer Vorteil der erfindungsgemässen Ausführung besteht darin, dass identisch ausgebildete Verankerungsteile sowohl für zementfreie Implantation als auch zur Implantation mittels Knochenzement verwendbar sind, wobei in der letzteren Anwendung insbesondere die im Bereich des Knochenzementbetts befindlichen Durchtrittsöffnungen durch die Verschlussstopfen abgeschirmt werden und damit ein Kontakt zwischen dem Knochenzement und der Gelenkauflage verhindert wird. Der erfindungsgemäss bestückte Verankerungsteil ermöglicht ferner die Verwendung einer vorteilhaft einfach ausgeführten Gelenkauflage in einer von der Anordnung der Verschlussstopfen vollständig unabhängigen Formgebung.

Ausgestaltungen des Erfindungsgegenstandes sind in den abhängigen Patentansprüchen angegeben.

Eine mit einer erfindungsgemässen Tibiaplattform ausgestattete Kniegelenkprothese ist Gegenstand des Patentanspruchs 8.

Ein Verschlussstopfen für eine erfindungsgemässe Tibiaplattform ist Gegenstand des Patentanspruchs 9.

Die Erfindung wird anhand eines in der Zeichnung schematisch dargestellten Ausführungsbeispiel erläutert.
Es zeigen:
- Fig.1: Teile einer erfindungsgemäss ausgebildeten Kniegelenkprothese mit einer Tibiaplattform in einem Schnitt entsprechend der Linie I - I in Fig.2,
- Fig.2: einen Teil der Tibiaplattform nach Fig.1 in einer Draufsicht,
- Fig.3: eine Einzelheit der Tibiaplattform nach Fig.1 in einer grösseren Darstellung, mit einem Verschlussstopfen in einem Schnitt entsprechend der Linie III - III in Fig.4,
- Fig.4: den Verschlussstopfen nach Fig. 2 in einer Untersicht gemäss Pfeil IV in Fig.3.

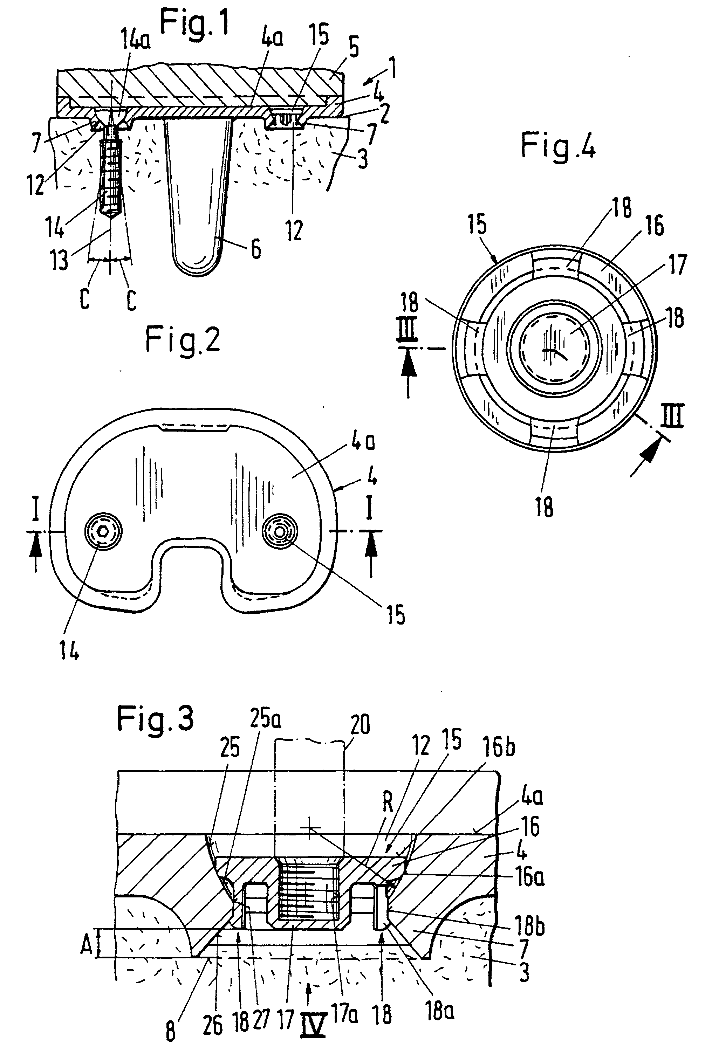

Die Kniegelenkprothese nach den Fig.1 und 2 enthält eine Tibiaplattform 1 und einen auf eine Resektionsfläche 2 eines Tibiaknochens 3 aufsetzbaren Verankerungsteil 4 aus Metall, z.B. Titan, und eine auf eine Stützfläche 4a des Verankerungsteils 4 aufsetzbare und mit diesem verklinkbare Gelenkauflage 5 aus einem Kunststoff, z.B. Polyäthylen, umfasst. Der Verankerungsteil 4 ist mit einem in eine Ausnehmung des Tibiaknochens 3 einführbaren zentralen Zapfen 6 und mit zwei in entsprechende Vertiefungen des Tibiaknochens 3 einführbaren ringförmigen Vorsprüngen 7 ausgeführt, welche je mit einer den Verankerungsteil 4 durchsetzenden Durchtrittsöffnung 12 versehen sind. Die Durchtrittsöffnungen 12 sind je zur Aufnahme eines im Tibiaknochen 3 verankerbaren Befestigungselementes 14 beliebiger Art, darstellungsgemäss in Form einer in das Knochengewebe einschraubbaren Knochenschraube, geeignet bzw. bestimmt. Zur Befestigung des Verankerungsteils 4 am Tibiaknochen 3 ist beim dargestellten Beispiel ein einziges Befestigungselement 14 vorgesehen, welches hauptsächlich der primär stabilen Fixierung des Verankerungsteils 4 an der Resektionsfläche 2 dient. Die für die Befestigung nicht benutzte Durchtrittsöffnung 12 des Verankerungsteils 4 ist mit einem von oben, von der Femurseite her, einsetzbaren und feststellbaren Verschlussstopfen 15 versehen.

Nach einer anderen, nicht dargestellten Ausführungsform kan der Verankerungsteil 4 mehrere, z.B. vier Durchtrittsöffnungen 12 aufweisen, und es können zwei oder mehr, z.B. drei, entsprechende Befestigungselemente 14 vorgesehen sein, wobei die nicht benutzten, von Befestigungselementen 14 freien Durchtrittsöffnungen 12 ebenfalls je mit einem Verschlussstopfen 15 versehen sind. Es ist auch eine Ausführung möglich, bei der auf die Anordnung eines Befestigungselements 14 verzichtet und alle Durchtrittsöffnungen 12 mit Verschlussstopfen 15 versehen sind.

Entsprechend der Darstellung nach den Fig.1 und 3 sind die Durchtrittsöffnungen 12 je mit einer femurseitigen, oberen Ansenkung 25 und einer tibiaseitigen, unteren Ansenkung 26 ausgeführt, welche mit der oberen Ansenkung 25 eine örtlich verengte Umfangspartie 27 der Durchtrittsöffnung 12, und mit dem tibiaseitigen, unteren Endabschnitt des Vorsprungs 7 eine Randpartie 8 in Form einer in das Knochenbett einführbaren Schneidkante begrenzt. Wie insbesondere aus der Fig.2 hervorgeht, können die oberen Ansenkungen 25 der Durchtrittsöffnungen 12 je eine domartige Form aufweisen, die durch einen kegelförmigen oberen Endabschnitt und eine zwischen diesem und der Umfangspartie 27 ausgebildete, zumindest annähernd sphärische Stützfläche 25a bestimmt ist. Die Stützflächen 25a sind gemäss Fig.3 je mit einem Radius R ausgeführt. Gemäss Fig. 1 können die Befestigungselemente 14 je mit einer Kopfpartie 14a ausgeführt sein, welche eine an die konkave Stützfläche 25a anlegbare, entsprechend konvexe Aufsetzfläche aufweist und welche dementsprechend die Anbringung des Befestigungselementes 14 im Knochengewebe 1 jeweils in einer Winkelstellung zulässt, die derjenigen der Achse 13 der Durchtrittsöffnung 12 entspricht oder die von dieser Achse 13 um einen in Fig. 1 angedeuteten Winkel C abweicht. Die dargestellte Ausführung gestattet somit eine entsprechende, innerhalb dieses Winkelbereichs variable Anpassung an die jeweils gegebenen anatomischen Verhältnisse.

Der Verschlussstopfen 15 enthält einen in die obere Ansenkung 25 der jeweils nicht benutzten Durchtrittsöffnung 12 versenkbar einführbaren tellerartigen Dichtungsteil 16, eine von diesem gegen die Tibiaseite des Verankerungsteils 4 vorstehende, zentrale hutartige Erhebung 17 und eine Anzahl, darstellungsgemäss vier, von einer Randpartie des Dichtungsteils 16 gegen die Tibiaseite des Verankerungsteils 4 vorstehende, segmentartig angeordnete Biegefedern 18. Der Dichtungsteil 16 ist mit einer über seinen Umfang umlaufenden, an eine Wandpartie der Ansenkung 25, darstellungsgemäss an die Stützfläche 25a, anlegbaren Dichtfläche 16a ausgeführt. Der Verschlussstopfen 15 wird durch die Biegefedern 18 in der Durchtrittsöffnung 12 gehalten und mit der Dichtfläche 16a an die Stützfläche 25a angepresst.

Wie insbesondere aus der Fig.3 hervorgeht, kann die Dichtfläche 16a durch eine der Stützfläche 25a entsprechende konische Gegenfläche des Dichtungsteils 16 gebildet sein, welche mit der Stützfläche 25a über eine ringförmige Kontaktfläche zusammenwirkt und damit eine wirksame Abdichtung der Durchtrittsöffnung 12 gewährleistet. Die Erhebung 17 ist mit einer gegen die Femurseite des Verankerungsteils 4 offenen Sackbohrung 17a ausgeführt, die darstellungsgemäss mit einem Gewinde versehen sein kann und die zur Aufnahme eines Mitnehmerteils eines strichpunktiert angedeuteten Setzwerkzeugs 20 bestimmt ist. Die Biegefedern 18 sind durch aus dem Randbereich des Dichtungsteils 16 nach aussen abstehende, relativ zueinander bewegliche, elastisch verformbare laschenartige Wandsegmente gebildet, welche je einen in die untere Ansenkung 26 einführbaren, gegen deren Wand verspannbaren Endabschnitt 18a und eine der verengten Umfangspartie 27 der Durchtrittsöffnung 12 entsprechende, diese aufnehmende Vertiefung 18b aufweisen. Darstellungsgemäss ist der Verschlussstopfen 15 so ausgeführt, dass er in der Durchtrittsöffnung 12 in einem Abstand A von der Randpartie 8 gehalten ist, welcher ein Eindringen derselben um ein entsprechendes Mass in das Knochenbett zulässt.

Der Verschlussstopfen 15, der aus dem gleichen Material wie der Verankerungsteil 4, z.B. aus einer Titanlegierung, bestehen kann, kann jeweils durch das Setzwerkzeug 20 in die Durchtrittsöffnung 12 eingeführt werden, wobei die Biegefedern 18 beim Uebergang der Endpartien 18a über die Umfangspartie 27 elastisch verformt werden und anschliessend mit der Wand der Durchtrittsöffnung 12 eine Schnappverbindung bilden, durch welche der Dichtungsteil 16 mit der Dichtfläche 16a gegen die Stützfläche 25a verspannt und damit die im Bereich der Durchtrittsöffnung 12 befindliche Partie des Knochengewebes 1 gegen die Femurseite des Verankerungsteils 4 abgedichtet und ein Durchtritt von Abriebpartikeln der Gelenkauflage 5 verhindert wird. Der Verschlussstopfen 15 kann über das Setzwerkzeug 20 auf einfache Weise in die Durchtrittsöffnung 12 eingesetzt und aus dieser entfernt werden. Die beschriebene Ausführung des Verschlusstopfens 15 ermöglicht auch bei relativ dünnen Verankerungsteilen 4, z.B. solchen mit Wandstärken von 4 bis 5 mm, eine sichere Abdichtung der Durchtrittsöffnungen 12, welche in der Regel für eine Anbringung von Gewinden nicht geeignet sind.

Es sind zahlreiche Ausführungsformen der Erfindung möglich. Abweichend von der Darstellung nach Fig.3, kann der Verschlussstopfen 15 so ausgeführt sein, dass seine femurseitige Oberfläche 16b in eingeschnappter Stellung im wesentlichen bündig mit der Stützfläche 4a des Verankerungsteils 4 übereinstimmt, wodurch ein Kaltfliessen des Kunststoffs der Gelenkauflage 5 in diesem Bereich verhindert werden kann. Entsprechend kann der Verschlussstopfen 15 mit einer grösseren Höhenabmessung und/oder mit einem dickeren Dichtungsteil 16 ausgeführt sein. Der Dichtungsteil 16 kann auch mit einer anders geformten, z.B. zylindrischen oder sphärischen, Dichtfläche 16a ausgeführt sein, die zum Zusammenwirken mit der Stützfläche 25a oder mit dem kegeligen Endabschnitt 25 der Ansenkung bestimmt sein kann. Nach einer nicht dargestellten Ausführungsform können die oberen Ansenkungen der Durchtrittsöffnungen 12 je über die ganze Tiefe kegelförmig ausgeführt und zur Aufnahme eines entsprechend kegelförmigen Kopfteils eines Befestigungselementes oder einer Hülse bestimmt sein, die ein in unterschiedlichen Winkelstellungen anbringbares Befestigungselement aufnehmen kann. Auch bei einer derartigen Ausführung können die Verschlussstopfen 15 mit an die Ansenkungen 25 flächig anlegbaren kegelförmigen Dichtflächen oder gegebenenfalls mit unter Linienberührung anlegbaren, z.B. zylindrischen Dichtflächen ausgeführt sein. Anstelle der dargestellten Schraubverbindung zwischen der Erhebung 17 und dem Setzwerkzeug 20 kann auch eine andere Mitnehmerverbindung, z.B. eine lösbare Hakenverbindung oder dergleichen, vorgesehen sein. Es versteht sich ferner, dass entsprechende Verschlussstopfen auch je mit einer von der dargestellten Anordnung abweichenden Anzahl, z.B. drei, sechs, acht oder mehr, Biegefedern 18 in beliebiger Ausführung versehen sein können.

Zusammenfassend lässt sich die Erfindung wie folgt beschreiben:

Die Tibiaplattform enthält eine Gelenkauflage 5 und einen Verankerungsteil 4 mit Durchtrittsöffnungen 12 für Befestigungselemente 14 zum Befestigen des Verankerungsteils 4 am Tibiaknochen 3. Die Durchtrittsöffnungen sind je mit einer femurseitigen, oberen Ansenkung 25 ausgeführt. Mindestens eine, für die Befestigung unbenutzte Durchtrittsöffnung 12 ist mit einem von oben her einsteckbaren Verschlussstopfen 15 versehen, welcher mit einer in der Ansenkung 25 abstützbaren, über diese umlaufenden Dichtfläche 16a ausgeführt ist. Der Verschlusstopfen 15 enthält eine Anzahl in die betreffende Durchtrittsöffnung 12 einführbare Biegefedern 18, die mit der Wand der Durchtrittsöffnung 12 eine Schnappverbindung bilden, und eine hutartige zentrale Erhebung 17, welche mit einer nach oben offenen Sackbohrung 17a zur Aufnahme eines Setzwerkzeugs 20 versehen ist. Dadurch wird eine sichere Abdichtung der Durchtrittsöffnungen 12 erzielt und ein Austreten von gegebenenfalls zwischen dem Verankerungsteil 4 und der Gelenkauflage 5 entstehenden Abriebpartikeln in das Tibia-Knochenbett verhindert.

## Patentansprüche

1. Tibiaplattform zu einer Kniegelenkprothese mit einem zum Befestigen an einem Tibiaknochen (3) und zur Aufnahme einer Gelenkauflage (5) bestimmten Verankerungsteil (4), der Durchtrittsöffnungen (12) aufweist, welche je zur Aufnahme eines in den Tibiaknochen (3) einführbaren Befestigungselementes (14) zum Befestigen des Verankerungsteils (4) geeignet bzw. bestimmt sind und je mit einer an der Femurseite des Verankerungsteils (4) ausgebildeten Ansenkung (25) ausgeführt sind,
**dadurch gekennzeichnet, dass** bei Nichtbenutzung mindestens einer der Durchtrittsöffnungen (12) die nicht benutzte Durchtrittsöffnung (12) mit einem von der Femurseite her einsteckbaren Verschlussstopfen (15) versehen ist, welcher mit einer in der Ansenkung (25) abstützbaren, über diese umlaufenden Dichtfläche (16a) ausgeführt ist und welcher eine Anzahl in die Durchtrittsöffnung (12) einführbare Biegefedern (18) aufweist, die mit der Wand der Durchtrittsöffnung (12) eine Schnappverbindung bilden.

2. Tibiaplattform nach Anspruch 1, **dadurch gekennzeichnet, dass** die Ansenkungen (25) der Durchtrittsöffnungen (12) je eine domartige Form aufweisen, die durch eine zumindest annähernd sphärische Stützfläche (25a) für das Befestigungselement (14) bestimmt ist, und dass die Dichtfläche (16a) des Verschlussstopfens (15) an einer an diese sphärische Stützfläche (25a) anlegbaren, entsprechend konischen Umfangspartie des Verschlussstopfens (15) ausgebildet ist.

3. Tibiaplattform nach Anspruch 1 oder 2, **dadurch gekennzeichnet, dass** der Verschlussstopfen (15) eine im Abstand von den Biegefedern (18) angeordnete, gegen die Tibiaseite des Verankerungsteils (4) vorstehende, hutartige zentrale Erhebung (17) aufweist, welche mit einer gegen die Femurseite offenen Sackbohrung (17a) zur Aufnahme eines in diese einführbaren Mitnehmerteils eines Setzwerkzeugs (20) versehen ist.

4. Tibiaplattform nach Anspruch 3, **dadurch gekennzeichnet, dass** die Sackbohrung (17a) mit einem Gewinde versehen ist.

5. Tibiaplattform nach einem der vorangehenden Ansprüche, **dadurch gekennzeichnet, dass** die Biegefedern (18) durch aus dem Bereich der Dichtfläche (16a) gegen die Tibiaseite abstehende, relativ zueinander bewegliche laschenartige Wandsegmente des Verschlussstopfens (15) gebildet sind.

6. Tibiaplattform nach einem der vorangehenden Ansprüche, **dadurch gekennzeichnet, dass** die Durchtrittsöffnungen (12) je mit einer an der Tibiaseite des Verankerungsteils (4) ausgebildeten, zweiten Ansenkung (26) ausgeführt sind, welche mit der femurseitigen, ersten Ansenkung (25) eine örtlich verengte Umfangspartie (27) der Durchtrittsöffnung (12) begrenzt, und dass die Biegefedern (18) des Verschlussstopfens (15)je mit einem in die zweite Ansenkung (26) einführbaren Endabschnitt (18a) und mit einer der verengten Umfangspartie (27) entsprechenden Vertiefung (18b) ausgeführt sind.

7. Tibiaplattform nach einem der vorangehenden Ansprüche, mit Durchtrittsöffnungen (12), die je an einem in den Tibiaknochen (3) einführbaren Vorsprung (7) des Verankerungsteils (4) ausgebildet sind,
**dadurch gekennzeichnet, dass** die Durchtrittsöffnungen (12) je mit einem tibiaseitigen Endabschnitt des betreffenden Vorsprungs (7) eine Randpartie (8) nach Art einer Schneidkante begrenzen, und dass der Verschlussstopfen (15) in einem Abstand (A) von dieser Randpartie (8) gehalten ist, welcher ein Eindringen der Randpartie (8) in das Knochengewebe zulässt.

8. Kniegelenkprothese mit einer Tibiaplattform nach einem der vorangehenden Ansprüche.

9. Verschlussstopfen für eine Tibiaplattform, welcher mit einer umlaufenden Dichtfläche (16a) versehen ist, die in einer Ansenkung (25) einer Tibiaplattform abstützbar ist, und welcher eine Anzahl Biegefedern (18) aufweist, die in eine Durchtrittsöffnung (12) einer Tibiaplattform einführbar sind und die so ausgebildet sind, dass sie mit der Wand der Durchtrittsöffnung (12) eine Schnappverbindung bilden können.

## Claims

1. Tibia platform for a knee joint prosthesis with an anchoring part (4) for fastening to a tibia bone (3) and for receiving a joint support (5), the anchoring part (4) having passage openings (12) which are suited and/or designed to receive one fastening element (14) each which can be introduced into the tibia bone (3) for fastening the anchoring part (4) and are each constructed with a countersink (25) on the femur side of the anchoring part (4),
**characterised in that** when at least one of the passage openings (12) is not used, the unused passage opening (12) is provided with a closure plug (15) which can be inserted from the femur side and is constructed with a sealing surface (16a) which can be supported in one of the countersinks (25) so as to extend around it, the closure plug having a number of spring tongues (18) which can be inserted into the passage opening (12) which form a snap connection with the wall of the passage opening (12).

2. Tibia platform in accordance with claim 1 **characterised in that** the countersinks (25) of the passage openings (12) each have the form of a dome which is determined by an at least approximately spherical supporting surface (25a) for the fastening element (14); and **in that** the sealing surface (16a) of the closure plug (15) is formed on a correspondingly conical peripheral part of the closure plug (15) which can contact this spherical supporting surface (25a).

3. Tibia platform in accordance with claim 1 or 2 **characterised in that** the closure plug (15) has a hat-like central raised portion (17) placed at a distance from the spring tongues (18) protruding from the tibia side of the anchoring part (4), the raised part having a blind bore (17a) open towards the femur side for receiving a driver part of a setting tool (20) which can be inserted therein.

4. Tibia platform in accordance with claim 3 **characterised in that** the blind bore (17a) is provided with a thread.

5. Tibia platform in accordance with one of the preceding claims **characterised in that** the spring tongues (18) are formed of lug-like wall sections of the closure plug (15) protruding from the vicinity of the sealing surface (16a) toward the tibia side and movable relative to one another.

6. Tibia platform in accordance with one of the preceding claims **characterised in that** each of the passage openings (12) is constructed with a second countersink (26) formed at the tibia side of the anchoring part (4), which together with the first, femur-side countersink (25) bounds a locally narrowed peripheral part (27) of the passage opening (12); and **in that** the spring tongues (18) of the closure plug (15) are each constructed with an end section (18a) which can be inserted into the second countersink (26) and with a depression (18b) corresponding to the narrowed peripheral part (27).

7. Tibia platform in accordance with one of the preceding claims with passage openings (12) which are each formed at one projection (7) of the anchoring part (4) which can be introduced into the tibia bone (3) **characterised in that** a tibia-side end section of the relevant projection (7) of each of the passage openings (12) bounds an edge portion (8) in the manner of a cutting edge; and **in that** the closure plug (15) is held at a distance (A) from this edge portion (8), which allows a penetration of the edge portion (8) into the bone tissue.

8. Artificial knee joint with a tibia platform in accordance with one of the preceding claims.

9. Closure plug for a tibia platform which is provided with a sealing surface (16a) extending around its periphery which can be supported in a countersink (25) of a tibia platform and which has a plurality of spring tongues (18) which can be inserted into a passage opening (12) of a tibia platform and which are formed such that they can form a snap connection with the wall of the passage opening (12).

## Revendications

1. Plateau de tibia pour une prothèse d'articulation du genou avec une partie d'ancrage (4) destinée à la fixation à un os de tibia (3) et à la réception d'un appui d'articulation (5), qui présente des ouvertures de passage (12) qui sont appropriées respectivement prévues chacune pour la réception d'un élément de fixation (14) insérable dans l'os de tibia (3) pour la fixation de la partie d'ancrage (4) et qui sont réalisées chacune avec un chanfrein (25) réalisé au côté fémur de la partie d'ancrage (4), **caractérisé en ce que** lors de la non-utilisation au moins d'une des ouvertures de passage (12), l'ouverture de passage (12) non utilisée est pourvue d'un bouchon de fermeture (15) insérable depuis le côté fémur, qui est réalisé avec une face d'étanchéité (16a) s'appuyant dans le chanfrein (25), s'étendant sur celui-ci et qui présente un nombre de ressorts élastiques (18) insérables dans l'ouverture de passage (12) qui forment avec la paroi de l'ouverture de passage (12) une liaison à enclenchement.

2. Plateau de tibia selon la revendication 1, **caractérisé en ce que** les chanfreins (25) des ouvertures de passage (12) ont chacun une forme semblable à un dôme qui est prévu par une face d'appui (25a) au moins approximativement sphérique pour l'élément de fixation (14), et **en ce que** la face d'étanchéité (16a) du bouchon de fermeture (15) est réalisée à une partie périphérique conique correspondante du bouchon de fermeture (15) applicable à cette face d'appui sphérique (25a).

3. Plateau de tibia selon la revendication 1 ou 2, **caractérisé en ce que** le bouchon de fermeture (15) présente une surélévation centrale (17) en forme de chapeau disposée à une distance des ressorts élastiques (18), faisant saillie vers le côté tibia de la partie d'ancrage (4), qui est pourvue d'un trou borgne (17a) ouvert vers le côté fémur pour la réception d'une partie d'entraînement d'un outil de pose (20) insérable dans celui-ci.

4. Plateau de tibia selon la revendication 3, **caractérisé en ce que** le trou borgne (17a) présente un filetage.

5. Plateau de tibia selon l'une des revendications précédentes, **caractérisé en ce que** les ressorts élastiques (18) sont formés par des segments de paroi en forme de languette du bouchon de fermeture (15), faisant saillie de la zone de la face d'étanchéité (16a) vers le côté tibia, déplaçables les uns relativement aux autres.

6. Plateau de tibia selon l'une des revendications précédentes, **caractérisé en ce que** les ouvertures de passage (12) sont réalisées chacune avec un deuxième chanfrein (26) réalisé au côté du tibia de la partie d'ancrage (4) qui délimite avec le premier chanfrein (25) du côté du fémur une partie périphérique (27) localement rétrécie de l'ouverture de passage (12), et **en ce que** les ressorts élastiques (18) du bouchon de fermeture (15) sont réalisés chacun avec un tronçon d'extrémité (18a) insérable dans le deuxième chanfrein (26) et avec un creux (18b) correspondant à la partie de pourtour rétrécie (27).

7. Plateau de tibia selon l'une des revendications précédentes, avec des ouvertures de passage (12) qui sont réalisées respectivement à une saillie (7) de la partie d'ancrage (4) insérable dans l'os de tibia (3), **caractérisé en ce que** les ouvertures de passage (12) délimitent chacune avec un tronçon d'extrémité, côté tibia, de la saillie concernée (7) une partie de bord (8) à la manière d'une arête de coupe, et **en ce que** le bouchon de fermeture (15) est tenu à un écart (A) de cette partie de bord (8), qui permet une pénétration de la partie de bord (8) dans le tissu osseux.

8. Prothèse d'articulation du genou avec un plateau de tibia selon l'une des revendications précédentes.

9. Bouchon de fermeture pour un plateau de tibia, qui est pourvu d'une face d'étanchéité (16a) s'étendant tout autour qui peut s'appuyer dans un chanfrein (25) d'un plateau de tibia et qui présente un nombre de ressorts élastiques (18) qui sont insérables dans une ouverture de passage (12) d'un plateau de tibia et qui sont réalisés de telle manière qu'ils peuvent former avec la paroi de l'ouverture de passage (12) une liaison à enclenchement.
